# EUROPEAN PATENT APPLICATION

(11) **EP 0 535 283 A1**
(43) Date of publication of application: **07.04.1993**
(21) Application number: 91402630.7
(22) Date of filing: 02.10.1991
(51) Int. Cl.: C07F 9/655

(54) **Cardioprotective tocopherol analogs**

(71) Applicant: MERRELL DOW PHARMACEUTICALS INC., Cincinnati Ohio 45215-6300 (US)
(72) Inventor: Grisar, J. Martin, F-67160 Wissembourg (FR); Petty, Margaret A., F-67000 Strasbourg (FR); Bolkenius, Frank, W-7640 Kehl (DE)
(74) Representative: Gillard, Marie-Louise

(57) **Abstract**

This invention relates to alkylated phosphonium alkylene derivatives of certain 2H-1-benzopyrans, to the intermediates and processes useful for their preparation, to their free-radical scavenger and cardioprotective properties and to their end-use application as therapeutic agents.

## Description

This invention relates to alkylated phosphonium alkylene derivatives of certain 2H-1-benzopyrans, to the intermediates and processes useful for their preparation, to their free-radical scavenger and cardioprotective properties and to their end-use application as therapeutic agents.

More specifically this invention relates to alkylated phosphonium alkylene derivatives of the formula 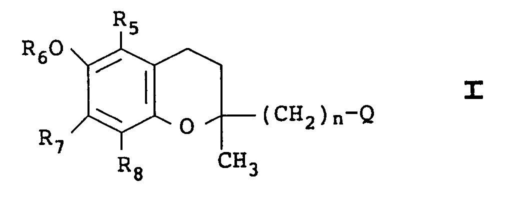
the (R) and (S) enantiomers and racemic mixtures thereof, and the pharmaceutically acceptable salts thereof wherein
- Q: is P^{⊕}R₁R₂R₃·X^{⊖},
- X: is a halide or OS(O)₂R₄, with R₄ being H, C₁₋₆ alkyl, aryl or aralkyl,
- R₁, R₂ and R₃: each individually are a C₁₋₆ lower alkyl, phenyl, benzyl or phenethyl,
- R₅: is H or C₁₋₆ alkyl,
- R₆: is H or -C(O)R, R being H or C₁₋₉ alkyl,
- R₇: is H or C₁₋₆ alkyl,
- R₈: is H or C₁₋₆ alkyl and n is an integer of 1 to 6.

As used herein, the moiety (CH₂)ₙ of Formula I wherein n is an integer of one to six represents a C₁₋₆ straight or branched-chain alkylene including such preferred species as methylene, ethylene, propylene, t-butylene, n-butylene, n-hexylene and isopropylene. The term "C₁₋₆ alkyl" includes the straight and branched-chain radicals having up to six carbon atoms with methyl, ethyl, propyl, n-butyl, t-butyl, pentyl and hexyl being representative. The term "-C(O)R" includes those acyl moieties wherein R is H and C₁₋₉ alkyl embracing formyl and the straight and branched-chain alkylcarbonyl moieties having up to ten carbon atoms including methylcarbonyl, ethylcarbonyl, propylcarbonyl, t-butylcarbonyl and n-hexylcarbonyl as preferred representatives. When used, aryl preferably is phenyl or phenyl substituted with C₁-C₆ alkyl radicals (e.g. toluene) and aralkyl is benzyl or phenethyl, the phenyl moiety of each optionally bearing C₁-C₆ lower alkyl radicals.

The moiety "Q" includes those quaternary phosphonium derivatives attached to the alkylene moiety wherein P^{⊕}R₁R₂R₃·X^{⊖} each of R₁, R₂ and R₃ may be a C₁₋₆ alkyl radical, phenyl, benzyl or phenethyl. Although it is preferred to have the R₁, R₂ and R₃ radicals the same, this invention includes those derivatives wherein the R₁, R₂ and R₃ radicals are different. Preferably these radicals are methyl, ethyl and phenyl.

The term "pharmaceutically acceptable acid addition salts" embraces those salts capable of being formed by the interaction of an organic or inorganic acid with a pharmaceutical base compound to yield a non-toxic pharmaceutically acceptable entity. Illustrative inorganic acids which form suitable salts include hydrochloric, hydrobromic, sulphuric and phosphoric acid and acid metal salts such as sodium monohydrogen orthophosphate and potassium hydrogen sulfate. Illustrative organic acids which form suitable salts include the mono-, di- and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, 2-phenoxybenzoic and sulfonic acids such as methane sulfonic acid, 4-methylbenzenesulfonic acid and 2-hydroxyethane sulfonic acid. Either the mono- or the di-acid salts can be formed, and such salts can exist in either a hydrated or a substantially anhydrous form. In general, salts of these compounds are crystalline materials which are soluble in water and various hydrophilic organic solvents.

In general the compounds of Formula I may be prepared by standard chemical processes and techniques analogously known in the art. In practice, the preparation of the compounds of Formula I conveniently utilizes 3,4-dihydro-2H-1-benzopyran-2-ols as starting materials which, for the most part, are known compounds. In those instances wherein any specific starting material is not known then such compounds may readily be prepared using the standard procedures analogously known in the art as well as by applying such processes as would be reasonably expected to produce the desired starting materials.

The preparation of the 3,4-dihydro-2,5,7,8-tetraalkyl-2H-1-benzopyran-2-ols and their conversion to the final products of Formula I is depicted in the following reaction schemes.

### Preparation of Intermediates

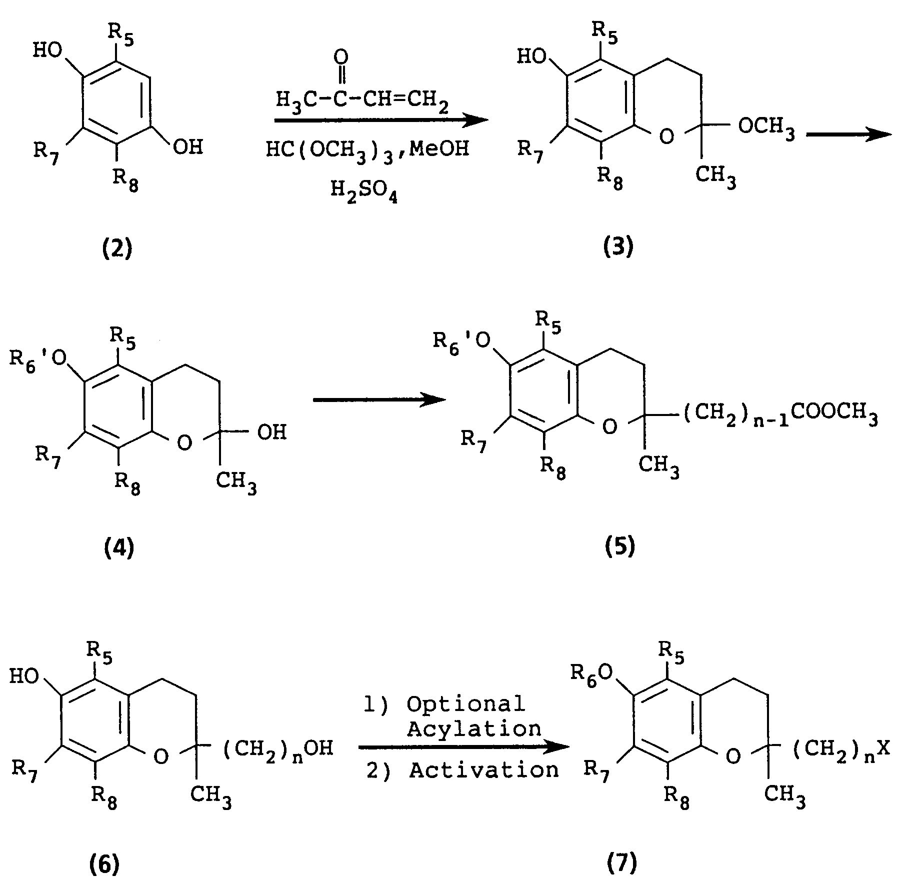

### Preparation of final compounds

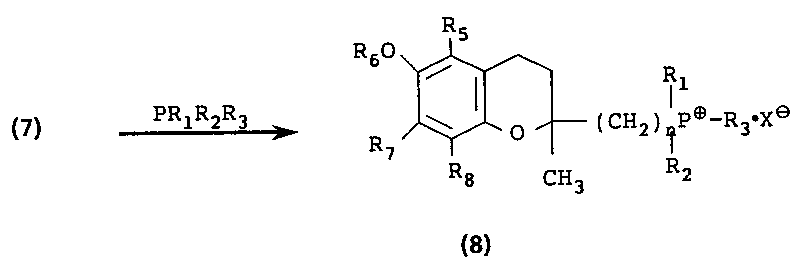

wherein n, R₁, R₂,R₃, R₅, R₆, R₇, R₈ and X are as previously defined and R₆' is -C(O)R, R being as previously defined.

The preparation of the intermediates start with the condensation of hydroquinones **(2)** with 3-butene-2-one in the presence of an acid, preferably sulfuric acid, the condensation being effected in methanol and trimethyl orthoformate. The so-produced dihydrobenzopyrans **(3)** are then sequentially subjected to acylation and hydrolysis reactions according to standard procedures to yield the hemiketals of Formula **(4)**. Introduction of the hydroxyalkyl moiety at the 2-position of the compounds of Formula **(4)** can be effected by Wittig or Horner type reactions, preferably by reaction of the compounds of Formula **(4)** with a trimethylphosphonoester (e.g. trimethylphosphonoacetate) to yield the esters of Formula **(5)** which are hydrolyzed, and then reduced (preferably with lithium aluminum hydride) to yield the alcohols of Formula **(6)**. These alcohols may also be formed directly by an acid catalyzed condensation of the hydroquinones **(2)** with the appropriate vinyl diols of Formulae **(10)** and **(11)**. 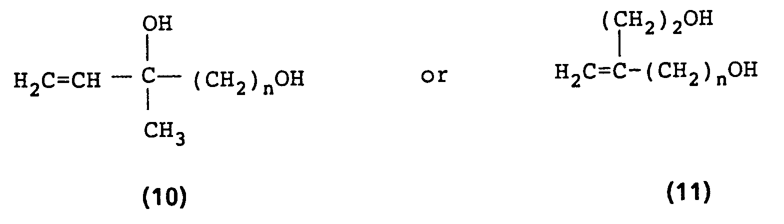
n being as defined above.

Prior to phosphonation, the alcohols of Formula **(6)** are first activated by converting the 2-position hydroxyalkyl moieties to either their halides or tosylates (i.e., X is a halide or a p-toluenesulfonyloxy radical of the formula - OS(O)₂R₄ wherein R₄ is as previously defined) according to standard conditions such as for example reaction of the alcohols with bromotriphenylphosphonium bromide (Ø₃PBr⁺Br⁻) obtained by reaction of triphenylphosphine with bromine in dichloromethane, or by reacting the alcohols with the appropriate sulfonyl halide (e.g., p-toluenesulfonyl chloride) in the presence of a base according to standard procedures well known in the art. The activated reactants (halides or tosylates) **(7)** may be converted to the desired quaternary phosphonium derivatives either before or after acylation of the 6-OH moiety. Standard procedures may be utilized to prepare the desired quaternary phosphoniums of Formula I. Standard procedures well known in the art may be used in the preparation of the quaternary phosphonium derivatives of Formula I. For example, reacting the activated compounds of Formula **(7)** with equimolar quantities of the appropriate trialkyl phosphine, under pressure, at temperatures of about 90°C to 150°C, in an inert solvent, preferably butanone, may be efficiently utilized. In the event that the tertiary phosphines are available (as free bases) the quaternary phosphonium derivatives may be prepared by reacting the tertiary phosphine with the appropriate alkyl halide or alkyl sulfonate (i.e., R₃X wherein X is a halide or alkyl sulfonate - OS(O)₂R₄) according to standard procedures such as by heating the reactants, preferably at reflux temperatures, in a polar solvent, preferably acetonitrile. As stated, it is possible to acylate the 6-position hydroxy moiety prior to phosphonation to obtain the O-acyl derivatives but the procedure is not preferred.

Further, as there is an asymmetric carbon atom at the 2-position, the compounds may occur as either the R- or the S-enantiomers, or mixtures thereof. The preparation of the individual enantiomeric form may be effected by resolving the acids of Formula **(5)** by standard and conventional means such as, for example, via the use of diastereomeric salts with optically active amines, or alternatively, by resolving the alcohols **(7)** as esters with optically active acids, e.g. L-2,4-MeClC₆H₃CHMeCOOH (Me representing methyl).

The following examples will serve to illustrate the techniques and processes described herein.

### EXAMPLE 1

### 3,4-Dihydro-2-(2-bromoethyl)-2,5,7,8-tetramethyl-2H-1-benzopyran-6-ol

To 11.0 g (0.042 mol) of triphenylphosphine in 200 ml of dichloromethane is added dropwise a solution of 6.71 g (0.042 mol) of bromine in 50 ml of dichloromethane. The solution is stirred for 30 min at room temperature, then 10.0 g (0.04 mol) of 3,4-dihydro-6-hydroxy-2,5,7,8-tetramethyl-2H-1-benzopyran-2-ethanol (CAS 79907-48-5) is added. The resulting solution is refluxed for 4 hours, allowed to cool overnight, washed with a solution of 15 g of sodium carbonate in 200 ml of water, dried over anhydrous sodium sulfate, filtered and evaporated. The resulting oil is crystallized from methanol to give 9.22 g of 3,4-dihydro-2-(2-bromoethyl)-2,5,7,8-tetramethyl-2H-1-benzopyran-6-ol.

The optically active enantiomers are obtained by substituting racemic 3,4-dihydro-6-hydroxy-2,5,7,8-tetramethyl-2H-1-benzopyran-2-ethanol with enantiomer R- (CAS 94425-68-0) or S- (CAS 94425-67-9) and by following the procedures of this example for each individual isomer.

### EXAMPLE 2

### 3,4-Dihydro-2-(2-bromoethyl)-2,5,7,8-tetramethyl-2H-1-benzopyran-6-yl acetate

To a solution of 9.22 g (0.029 mol) of 3,4-dihydro-2-(2-bromoethyl)-2,5,7,8-tetramethyl-2H-1-benzopyran-6-ol in 60 ml of lutidine is added 30 ml of acetic anhydride. The resulting solution is stirred at room temperature overnight. Water (30 ml) is added and some ice to keep the temperature around 30°C, the mixture is stirred for 30 min, more water and ice are added, the resulting precipitate is collected, washed with water and dried over phosphorus pentoxide under reduced pressure to give 10.0 g of powder. Recrystallization from a mixture of ethyl ether and pentane gives 9.41 g of 3,4-dihydro-2-(2-bromoethyl)-2,5,7,8-tetramethyl-2H-1-benzopyran-6-yl acetate, m.p. 102-103°C. Utilizing the R- and the S- enantiomers, as prepared in Example 1, the corresponding enantiomers are similarly prepared by following the procedure of this example.

### EXAMPLE 3

### [2-(3,4-Dihydro-6-hydroxy-2,5,7,8-tetramethyl-2H-1-benzopyran-2-yl)ethyl]-trimethylphosphonium bromide

A mixture of 3.13 g of 3,4-dihydro-2-(2-bromoethyl)-2,5,7,8-tetramethyl-2H-1-benzopyran-6-ol, 20 ml of a 1M solution of trimethylphosphine in tetrahydrofuran and 25 ml of 2-butanone is heated in a closed stainless steel vessel with stirring to 100°C for 44 hours. The vessel is cooled, opened, and its contents are transferred to a flask and evaporated to dryness. Two recrystallizations from acetonitrile give 3.0 g of the title compound, m.p. 230-233°C identified by elemental analysis, IR, UV and ¹H and ¹³C NMR spectra. The R- and S- enantiomers may similarly be produced.

### EXAMPLE 4

### 2-(3,4-Dihydro-2,5,7,8-tetramethyl-6-methylcarbonyloxy-2H-1-benzopyran-2-yl)ethyl-trimethylphosphonium bromide

Using equivalent amounts of the 6-acetate described in Example 2 in place of the 3,4-dihydro-2-(bromoethyl)-2,5,7,8-tetramethyl-2H-1-benzopyran-6-ol and by substantially following the procedure of Example 3, there is produced the title compound of this example.

Similarly by utilizing the R- and the S- enantiomers produced by Examples 1 and 2 and by following the procedure of this example there is produced the corresponding R- and S- enantiomers of the title compound of this example.

### EXAMPLE 5

### [2-(3,4-Dihydro-6-hydroxy-2,5,7,8-tetramethyl-2H-1-benzopyran-2-yl)ethyl]-triethylphosphonium bromide

Following the procedure described in Example 3, but using 1M triethylphosphine gives the title compoud, which is recrystallized from a mixture of acetonitrile and ethyl acetate, m.p. 187-188°C, identified by elemental analysis, IR, UV¹H, ¹³C NMR spectra.

Similarly by substituting the triethylphosphine with equivalent quantities of:
a) tripropylphosphine,
b) triisopropylphosphine,
c) dimethylphenylphosphine,
d) dimethylbenzylphosphine, or
e) ethylmethylphenylphosphine and by following the procedures of Examples 3 and 4, there is produced:
the tripropylphosphonium bromide,
triisopropylphosphonium bromide,
dimethylphenylphosphonium bromide,
dimethylbenzylphosphonium bromide and the
ethylmethylphenylphosphonium bromide analogs of [2-(3,4-dihydro-6-hydroxy-2,5,7,8-tetramethyl-2H-1-benzopyran-2-yl)ethyl]-trimethylphosphonium bromide and of [2-(3,4-dihydro-2,5,7,8-tetramethyl-6-methylcarbonyloxy-2H-1-benzopyran-2-yl)ethyl]-trimethylphosphonium bromide, including their R- and S- individual enantiomers.

### EXAMPLE 6

### 2-(3,4-Dihydro-6-hydroxy-2,7,8-trimethyl-2H-1-benzo-pyran-2-yl)ethyl-trimethylphosphoonium bromide

Following the procedure described in Example 3, but using 3,4-dihydro-6-hydroxy-2,7,8-trimethyl-2H-1-benzopyran-2-ethanol (CAS 93600-70-5) as starting material, the title compound is obtained.

### EXAMPLE 7

### 2-(3,4-Dihydro-6-hydroxy-2,5,8-trimethyl-2H-1-benzo-pyran-2-yl)ethyl-trimethylphosphonium bromide

Following the procedure described in Example 3, but using 3,4-dihydro-6-hydroxy-2,5,8-trimethyl-2H-1-benzopyran-2-ethanol (CAS 93600-69-2) as starting material, the title compound is obtained.

### EXAMPLE 8

### 2-(3,4-Dihydro-6-hydroxy-2,5,7-trimethyl-2H-1-benzo-pyran-2-yl)ethyl-trimethylphosphonium bromide

Following the procedure described in Example 3, but using 3,4-dihydro-6-hydroxy-2,5,7-trimethyl-2H-1-benzopyran-2-ethanol (CAS 93600-68-1) as starting material, the title compound is obtained.

### EXAMPLE 9

### 3-(3,4-Dihydro-6-hydroxy-2,5,7,8-tetramethyl-2H-1-benzo-pyran-2-yl)propyl-trimethylphosphonium bromide

Following the procedure described in Examples 1 and 3, but using 3,4-dihydro-6-hydroxy-2,5,7,8-tetramethyl-2H-1-benzopyran-2-propanol (CAS 104568-57-2) as starting material, the title compound is obtained.

Also, by using the appropriate chloro or sulfonate activated reactants in place of the 3,4-dihydro-2-(2-bromoethyl)-2H-1-benzopyran reactants, the corresponding phosphonium chlorides and phosphonium sulfonates of the compounds of the foregoing Examples (3 to 9) may be produced.

Having described the scope of the compounds of this invention as well as the generic and specific methods for preparing said compounds, the following information describes the utility, and the methods therefor, of the compounds of this invention.

When the blood supply to parts of the heart muscle is blocked, a myocardial infarct (heart attack) results and the deprived muscle tissue dies with the result of permanent heart damage. If the blood supply can be re-established within hours after infarction, the heart muscle tissue remains viable and permanent damage can be reduced. This can be accomplished by surgical as well as pharmacologic (thrombolysis) procedures and these processes are known as reperfusion.

Reperfusion is now widely and successfully applied and it has been claimed that fatalities due to myocardial infarction can be reduced by 20-30%. However, reperfusion also poses problems. Oxygen-deprived (ischemic) tissue finds itself in an abnormal state and is vulnerable when suddenly exposed to oxygen-rich blood. This has been termed the "oxygen paradox" and leads to reperfusion damage in the form of cell death. It has been postulated that this damage is due to oxygen-derived free radicals and, in particular, to the superoxide radical, O₂⁻. Evidence for this hypothesis has been obtained in animal experiments. B.R. Lucchesi and coworkers showed that the enzyme superoxide dismutase, as well as the free radical scavenger N-(mercaptopropionyl)glycine reduce canine myocardial reperfusion injury (Cir. Res., 1984, 54, 277-285; J. Cardiovasc. Pharmacol., 1986, 8, 978-88; Fed. Proc., 1987, 46, 2413-21).

Vitamin E, i.e., α-tocopherol, a well known compound of the formula 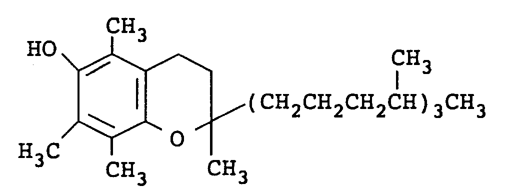
is a natural anti-oxidant that reacts with oxygen-derived free radicals as well as hydrogen peroxide. It has been shown that it is intercalated in lipid membranes and that its biological function is to protect biomembranes against oxidative attack. The anti-oxidant 3,4-dihydro-2,5,7,8-tetramethyl-2H-2-benzopyran-6-ol moiety of α-tocopherol is constantly regenerated by the ubiquitous cytosolic redox systems and for all practical purposes is a permanent membrane constituent that is constantly regenerated.

The compounds of this invention also possess a related or similar 3,4-dihydroxy-2,5,7,8-tetraalkyl-2H-1-benzopyran-2-yl moiety, but the 2-position lipophylic moiety of the α-tocopherol molecule, which is thought to be responsible for its ubiquitous incorporation into biomembranes, is replaced with a hydrophylic moiety to impart a greater affinity for cardiac tissue. Thus, the compounds of this invention are also useful as pharmacologic antioxidants and free radical scavengers and, in particular, as scavengers of superoxide anion radical O₂⁻. They can be therapeutically employed where reperfusion damage due to oxygen-derived free radicals and hydrogen peroxide causes cell death in tissues. This situation arises when total or partial blockade of blood supply to tissues is removed, either spontaneously (transient ischemia) or by pharmacologic or surgical intervention (thrombolysis, angioplasty, by-pass, organ transplant and the like). Tissues subjected to transient ischemia or reperfusion in various disease states, or by their medical treatment, are those of heart, lung, kidney, pancreas and brain. In particular, the now rapidly increasing practice of pharmacologic thrombolysis, also known as reperfusion, after coronary infarct and stroke, will benefit by prior or concomitant administration of a free radical scavenger such as the compounds of this invention. Similarly, surgical interventions, such as percutaneous transluminal coronary angioplasty, where a dilating balloon is used to increase the luminal diameter in severely occluded atherosclerotic vessels, coronary by-pass operations, and organ transplant surgery create conditions where reperfusion damage due to oxygen-derived radicals takes place and can be reduced by scavengers. Transient ischemia is one of the causative factors that lead to angina pectoris, and thus the compounds of this invention are also useful as antianginal agents.

The process of inflammation is also known to involve the release of superoxide radicals from phagocytic cells which cause some of the symptoms of rheumatoid arthritis and a free radical scavenger, such as the compounds of this invention, is also useful in the treatment of this disease. Of particular use of this anti-inflammatory use of the compounds if this invention is in the treatment of inflammatory lower bowel disease. The compounds may also be useful in the treatment of cancers and of aging since oxygen-derived free radicals have been identified among causative factors. For reviews, see B. Halliwell and C. Gutteridge, Biochem. J., 1984, 219, 1-14; TINS 1985, 22-6.

*In vitro* and *in vivo* activity for the compounds of this invention may be determined by the use of standard assays which demonstrate the free radical scavenging property, affinity for cardiac tissue and cardioprotective properties, as well as by comparison with agents known to be effective for these purposes.
Exemplary of the assay useful for determining the IC₅₀ values of the free-radical scavenging property of the compounds of this invention is by the *in vitro* inhibition of lipid per-oxidation in rat brain homogenates and the ID₅₀ values may be determined by the inhibition of *ex vivo* lipid peroxidation in mouse heart homogenates by known methodology. Affinity for cardiac tissue may be determined from the ratio of the IC₅₀/ID₅₀ values as well as other supplementary assays.

Most preferably, the compounds are administered intravenously particularly under crisis situations wherein it is essential that the therapeutic agent be gotten to its site of action as quickly as possible, such as in those emergency conditions caused by coronary infarction, stroke and surgical interventions, conditions which can cause severe reperfusion damage.

The compounds of this invention can be utilized both prophylactically and therapeutically. The amount of active ingredient for therapeutic administration can vary over a wide range and is dependent upon such factors as the species of mammal to be treated, its age, health, sex, weight, nature and the severity of the condition being treated. Generally, a therapeutically effective amount of the active ingredient to be administered will range from about 0.1 mg/kg to 10 mg/kg of body weight per day. For prophylactic administration, corresponding lower doses can be utilized.

The compounds of this invention can also be orally administered, preferably using more active ingredient per day than when parenterally administered, preferably taking divided doses 3 to 4 times per day. Preferably, enteral administration in post "crisis" situations, particularly after release from hospitalized conditions. The compounds can be used in standard dosage unit forms such as tablets, capsules, dragees, lozenges, elixirs, emulsions, suspensions, and in cases wherein topical application is preferred by suppository or sub-lingual administration. Tablets and capsules containing from 25 to 400 mg of active ingredient are preferred modes of enteral administration. Of course, in the treatment of inflammation the preferred method of administration is by depot injection directly to the situs of the inflammation area with follow-up enteral means of administration.

In preparing solid dose forms such as tablets, the active ingredient is generally blended with conventional pharmaceutical carriers or excipients such as gelatin, various starches, lactose, calcium phosphate or powdered sugar. The term pharmaceutical carrier as used herein also includes lubricants employed to improve the flow of tablet granulations and which prevent adhesion of tablet material to the surfaces of tablet dies and punches. Suitable lubricants include, for example, talc stearic acid, calcium stearate, magnesium stearate and zinc stearate. Also included within the definition of a pharmaceutical carrier as used herein, are disintegrating agents added to assist the break-up and dissolution of tablets following administration, as well as stabilizers (e.g., ascorbic acid), coloring and/or flavoring agents to enhance the qualities of the tablets.

Suitable liquid excipients for the preparation of liquid dosage unit forms include water and alcohols such as ethanol, benzyl alcohol and the polyethylene glycols, either with or without the addition of a surfactant. In general, the preferred liquid excipients, particularly for injectable preparations, include water, physiological and saline solutions, dextrose and glycol solutions such as an aqueous propylene glycol or polyethylene glycol solutions. In order to minimize or eliminate irritation at the site of injection, such compositions may contain a non-ionic surfactant having a hydrophile-lipophile balance (HLB) of from about 12 to about 17. The quantity of surfactant in such formulations ranges from about 5 to 15% by weight. The surfactant can be a single component having the above-identified HLB, or a mixture of two or more components having the desired HLB. Illustrative of surfactants useful in parenteral formulations are the class of polyoxyethylene sorbitan fatty acid esters as, for example, sorbitan monooleate and the high molecular weight adducts of ethylene oxide with a hydrophobic base, formed by the condensation of propylene oxide with propylene glycol. In certain topical and parenteral preparations, various oils can be utilized as carriers or excipients. illustrative of such oils are mineral oils, glyceride oils such as lard oil, cod liver oil, peanut oil, sesame oil, corn oil and soybean oil. For insoluble compounds, suspending agents may be added as well as agents to control the viscosity, as for example, magnesium aluminum silicate or carboxymethylcellulose. In addition to these excipients, buffers, preservatives and emulsifying agents may also be added.

The compounds of this invention can also be administered topically. This can be accomplished by simply preparing a solution of the compound to be administered, preferably using a solvent known to promote transdermal absorption such as ethanol or dimethyl sulfoxide (DMSO) with or without other excipients. Preferably topical administration will be accomplished using a patch either of the reservoir and porous membrane type or of a solid matrix variety.

Some suitable transdermal devices are described in U.S. Pat. Nos. 3,742,951, 3,797,494, 3,996,934, and 4,031,894. These devices generally contain a backing member which defines one of its face surfaces, an active agent permeable adhesive layer defining the other face surface and at least one reservoir containing the active agent interposed between the face surfaces. Alternatively, the active agent may be contained in a plurality of microcapsules distributed throughout the permeable adhesive layer. In either case, the active agent is delivered continuously from the reservoir or microcapsules through a membrane into the active agent permeable adhesive, which is in contact with the skin or mucosa of the recipient. If the active agent is absorbed through the skin, a controlled and predetermined flow of the active agent is administered to the recipient. In the case of microcapsules, the encapsulating agent may also function as the membrane.

In another device for transdermally administering the compounds in accordance with the present invention, the pharmaceutically active compound is contained in a matrix from which it is delivered in the desired gradual, constant and controlled rate. The matrix is permeable to the release of the compound through diffusion or microporous flow. The release is rate controlling. Such a system, which requires no membrane is described in U.S. Pat. No. 3,921,636. At least two types of release are possible in these systems. Release by diffusion occurs when the matrix is non-porous. The pharmaceutically effective compound dissolves in and diffuses through the matrix itself. Release by microporous flow occurs when the pharmaceutically effective compound is transported through a liquid phase in the pores of the matrix.

Of course, as is true in most instances wherein certain classes of chemical compounds have been found to have beneficial therapeutic end-use applications, certain sub-generic groups and certain specific compounds are preferred. In this instance the preferred compounds of Formula I are those wherein R₅, R₇ and R₈ are methyl; wherein R₆ is H, formyl, methyl carbonyl, t-butylcarbonyl, ethylcarbonyl, propylcarbonyl, pentylcarbonyl; wherein n is 2 (representing an ethylene moiety) and the substituents attached to the phosphorous atom are methyl or ethyl.

Of course, it is obvious that the 2-position methyl moiety may be removed or replaced with another lower alkyl (e.g., the 2-position methyl may be replaced with H, ethyl, propyl, butyl and the like). Such so-modified compounds are also contemplated within the scope of this invention for the utilities herein alleged, and may be prepared by standard procedures obvious to those skilled in the art.

## Claims

1. A compound of the formula 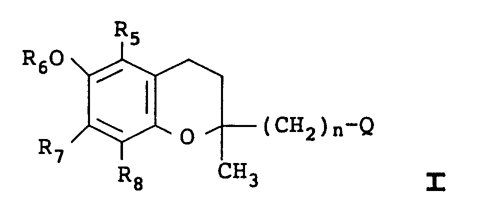 the (R) and (S) enantiomers and racemic mixtures thereof, and the pharmaceutically acceptable salts thereof wherein
Q is P^{⊕}R₁R₂R₃·X^{⊖},
X is a halide or OS(O)₂R₄, with R₄ being H, C₁₋₆ alkyl, aryl or aralkyl,
R₁, R₂ and R₃ each individually are a C₁₋₆ lower alkyl, phenyl, benzyl or phenethyl,
R₅ is H or C₁₋₆ alkyl,
R₆ is H or -C(O)R, R being H or C₁₋₉ alkyl,
R₇ is H or C₁₋₆ alkyl,
R₈ is H or C₁₋₆ alkyl and n is an integer of 1 to 6.
